(19) Europäisches Patentamt
European Patent Office
Office européen des brevets

(11) **EP 2 378 289 A1**

(12) **EUROPEAN PATENT APPLICATION**
published in accordance with Art. 153(4) EPC

(43) Date of publication:
**19.10.2011 Bulletin 2011/42**

(51) Int Cl.:
***G01N 33/543*** (2006.01)

(21) Application number: **10731166.4**

(22) Date of filing: **15.01.2010**

(86) International application number:
**PCT/JP2010/000198**

(87) International publication number:
**WO 2010/082497 (22.07.2010 Gazette 2010/29)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **15.01.2009 US 144910 P**

(71) Applicant: **ULVAC, INC.**
**Chigasaki-shi**
**Kanagawa 253-8543 (JP)**

(72) Inventors:
• **OZEKI, Tomomitsu**
**Chigasaki-shi**
**Kanagawa 253-8543 (JP)**

• **JITSUKAWA, Tomofumi**
**Chigasaki-shi**
**Kanagawa 253-8543 (JP)**
• **MITSUHASHI, Masato**
**Irvine, CA 92614 (US)**

(74) Representative: **Körber, Martin Hans**
**Mitscherlich & Partner**
**Patent- und Rechtsanwälte**
**Sonnenstraße 33**
**80331 München (DE)**

(54) **METHOD FOR MEASURING SYSTEMIC CONCENTRATION OF PROTEIN-CONTAINING MEDICAMENT**

(57)    It is an object of the invention to provide a method for rapid and accurate quantification of a sample in the human body of a patient.

The invention relates to a method for quantification of the body internal concentration of a protein-based drug, comprising step A comprising using a sensing device mounted with a substance binding to a protein contained in the protein-based drug through a specific interaction to determine the binding mass per unit area of a solution containing the protein-based drug to the substance mounted on the sensing device as a standard value and step B comprising assaying a collected biological sample by the sensing device and comparing the resulting value with the standard value to determine the concentration of the protein-based drug contained in the biological sample.

In such a manner, the blood concentration of a circulating protein-based drug can be quantified in patient bodies, to determine the optimal dose and enable an effective therapeutic treatment.

FIG. 1

EP 2 378 289 A1

## Description

Technical Field

The present invention relates to a method for rapid quantification of the body internal concentration of a protein-based drug and the complement-dependent Cytotoxicity activity thereof.

Background of the Invention

The invention claims the priority of a US provisional application No. 61/144, 910 filed on January 15, 2009, of which the contents are cited in the present description.
Doctors utilize various methods for determining a drug dose tailored to a patient (for example, patent reference 1). Insulin and antihypertensive drugs are prepared according to the physiological response of a patient to drugs including anticonvulsants and tranquilizers and various drug types. For some antibiotics, established blood concentrations exist, which function as standards for determining the doses of such antibiotics. In case that the blood concentration of a drug is below the minimal blood concentration established as effective for a disease, the dose thereof should be raised. In case that the blood concentration thereof is above the recommended level, a doctor reduces the dose so as to reduce the risk of adverse effects or to avoid the wasted use of the drug through simple prescription of an unnecessarily high dose of the drug.

A great number of antibody-based drugs have been developed in the last couple of years. Antibodies are proteins of specific amino acid sequences binding to antigens which are specific objects. The binding can inactivate the subjects, to mark the subjects for destruction by the immune system. In case that antibodies attach to biologically active substances (namely, chemotherapeutic agents or radioactive isotopes), the biologically active substances can effectively be attached to specific subjects. Although the use of monoclonal antibodies with cure effects has spread considerably, almost no effective method exists for monitoring the blood concentrations thereof or the effect of such drugs. For example, new drugs such as abciximab, rituximab, infliximab, adalimumab, and etanercept are monoclonal antibodies and bind to proteins in human bodies to inactivate specific proteins. The latter three drug types bind specifically to a tumor necrosis factor $\alpha$ (TNF$\alpha$). TNF$\alpha$ is a cytokine and is generally used in the immune system to destruct unnecessary cells and simultaneously suppress inflammation. The specific TNF$\alpha$ action has a relation with the pathogenesis of a great number of autoimmune diseases such as Crohn's disease, ankylosing spondylitis and rheumatoid arthritis. Via the reduction of the action of TNF$\alpha$, these drugs may possibly be effective therapeutic drugs of those diseases.

Unfortunately, therapeutic failures with the drug types described above often happen. By introducing antibodies into human bodies, the autoimmune system of a patient may exert a rejection to the drugs. Through the rejection, the amount of such drug binding to TNF$\alpha$ is decreased, to limit the therapeutic efficacy. Excessive anti-TNF$\alpha$ action is also problematic. Because these drugs are intended to inactivate the significant elements of the autoimmune system, consequently, there is a risk of infectious diseases forcing the suspension of the anti-TNF$\alpha$ therapy. Other influences of those drugs include possibilities of the lupus-like syndrome, exacerbated congestive heart failure, demyelination of nerve cells and the onset of hepatic toxicity. Doctors prescribing monoclonal antibody-based drugs essentially determine whether the dose of an anti-TNF$\alpha$ antibody is a sufficient amount to exert the efficacy thereof but is not a dose causing the occurrence of hazardous adverse actions.

The high cost of those monoclonal antibody-based drugs is the other reason why an appropriate dose thereof should importantly be determined. For example, the price of a single 100-mg infliximab dose exceeds 1,000 dollars. In case that the dose is far below the dose to exert the effect, not only the dose needs an enormous cost but also the dose simply falls into a wasted resource. In case that the dose is so excessive to involve the occurrence of adverse actions over the advantageous effect, the dose is simply a wasted resource and hazardous.

Unfortunately, a longer time is needed so as to determine the blood level of a monoclonal antibody drug, and the procedures are so complicated. The method generally employed is enzyme-linked immunosorbent assay (ELISA). Since the method is technically complicated and demands a very long time, the method is not suitable for rapid testing. Therefore, a method for objectively assaying the therapeutic effect as well as a simple rapid method for determining the blood concentration of the drug is needed.

In addition to the need of an ability to determine the blood concentration of an antibody-based drug, precise evaluation of the effect of the drug is essential. Autoimmune diseases involve the progress of various cell destructions including complex interactive actions of inflammatory mediators and complement-dependent cell damages. For example, the therapeutic method with infliximab is intended for the modulation of those processes. However, it is difficult to assay the effect of those drugs on abnormal immunoactivity. A report of subjective symptoms and a test with no specificity to inflammation are used for comparison with an ability to accurately assay the physiological reaction of a human body to a therapeutic method with a drug with specificity. So as to carefully and accurately administer a costly and risky antibody drug, the effect of such drug should necessarily be assayed accurately during the disease process.

Prior Technical References

Patent Reference

Patent Reference 1: JP-T-2003-535594

Summary of the Invention

Problem that the Invention is to Solve

It is therefore an object of the invention to provide a method for rapidly assaying a sample in the body of a patient appropriately.

Means for Solving the Problem

The invention relates to a method for assaying the body internal concentration of a protein-based drug and includes step A comprising using a sensing device mounted with a substance binding to the protein-based drug through a specific interaction to determine the binding mass per unit area of a solution containing the protein-based drug to the substance mounted on the sensing device as a standard value, and step B comprising assaying a collected biological sample by the sensing device and comparing the resulting value with the standard value to determine the concentration of the protein-based drug contained in the biological sample.

Advantages of the Invention

In accordance with the invention, the blood concentration of a circulating protein-based drug in the body of a patient can be assayed to determine the optimal dose of the drug and consequently establish an effective therapy with the drug.

Brief Description of the Drawings

Fig. 1 is a view depicting the concept of the inventive assay method.
Fig. 2 shows a graph depicting the specific binding of infliximab to the TNF$\alpha$ immobilized on the surface of the QCM sensor.
Fig. 3 shows graphs depicting the specific binding of various concentrations of infliximab in PBS and whole blood to the immobilized TNF$\alpha$.
Fig. 4 shows a graph depicting the binding of a complement-based protein to an infliximab-TNF$\alpha$ complex.

Modes for Carrying out the Invention

The inventive assay method includes a step A comprising preliminarily determining the binding mass per unit area of a solution containing a protein-based drug to a specifically binding substance on the surface of a sensing device as a standard value and a step B comprising assaying a collected biological sample itself by the sensing device to compare the resulting value with the standard value and determine the concentration of the protein-based drug contained in the biological sample.

The step A can be done, for example, by calculating or assaying the mass of a protein contained in the unit mass of a known protein-based drug solution. When a protein is contained in a buffer, for example, a method exists, comprising actually assaying the protein-based drug itself dissolved in the unit mass of the buffer solution by a sensing device described hereinafter. In case of a sensing device detecting the mass attached to the unit area, for example, the mass detected per the unit area is a standard value.

In accordance with the invention, a collected biological sample itself is detected directly by a sensing device, and when the biological sample is blood, for example, whole blood can be used for such assay, without needing any preliminary step for separating serum and the like, so that the assay can be done in a short time from the collection of blood to the assay. Furthermore, the collected biological sample requires no separation or the like, so that the sample of a small volume is simply needed, which reduces burdens to human bodies. Even if serum and plasma are used as biological samples, herein, the same assay value may be obtained.

In case that a biological sample is whole blood containing clot and serum, the whole blood is preferably added with an anti-coagulation agent. The reason is that coagulation during the assay can be prevented.

The step A is for the fixed standard value preliminarily assayed, and preferably there is no need for a testing person performing the step B to carry out the step A. For the improvement of the assay precision, herein, the step A is carried out simultaneously with the step B or is carried out within 24 hours before the step B.

As the sensing device for use in accordance with the invention, any of a quartz oscillator, a surface plasmon resonance device and an interferometer may be used.

The protein-based drug means a drug containing protein, which includes for example drugs containing proteins, such as monoclonal antibody, chimera monoclonal antibody, humanized monoclonal antibody, human monoclonal antibody and murine monoclonal antibody. Herein, the protein includes fusion proteins containing the antigen-binding sites of antibodies and fusion proteins containing the antigen-binding sites of receptors.

So as to detect a protein-based drug by the sensing device, a substance binding to a protein via a specific interaction is to be mounted on the sensing part of the sensing device. Specifically, a ligand binding to a protein through a specific interaction is immobilized on the sensing part of a sensing device. In such manner, the antibody-antigen binding of a protein-based drug and a ligand, and the ligand-receptor binding can be assayed as mass change and the like, on the sensing part.

Using the assay method, additionally, a complement-based protein interaction occurring via the binding of a monoclonal antibody-based drug as a protein-based drug to the sensing part of a sensing apparatus at an active state of a complement in a biological sample, is assayed together with an interaction of the monoclonal antibody-based drug at an inactive state of the complement in the biological sample, and based on the difference between the two assay values, it can be determined that the complement level is high in a manner corresponding to the cytotoxicity.

The recovery of the standard value and the comparison of the concentration as described above can be done with a known computer, and a printing or displaying apparatus outputting the results.

Examples

Examples of the invention are now described below with reference to drawings.

[Preparation and Explanation of Sensing Device]

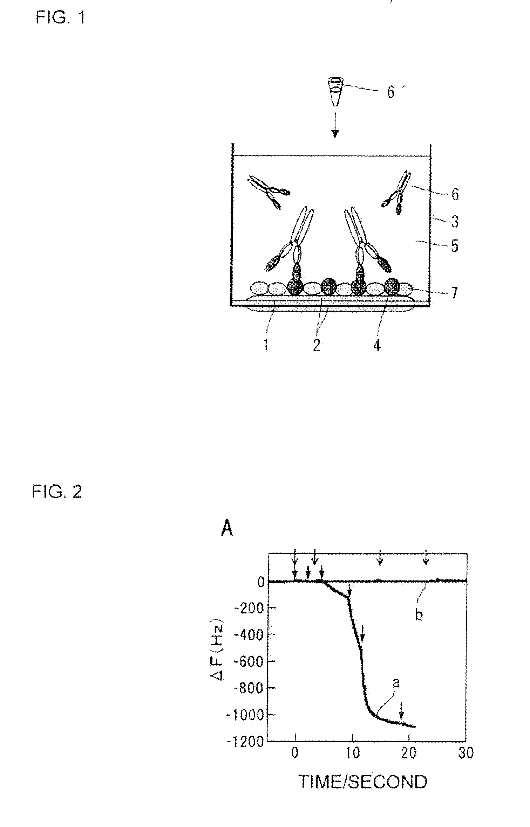

In the following Example, a biosensor utilizing QCM assaying mass per unit area was used for assay. As shown in Fig. 1, the biosensor is of a structure with gold electrodes 2, 2 on both the surfaces of a quartz plate 1. The structure is known. Arranging the biosensor on the bottom of a container 3, the resulting container is used as a cell. Not shown in the figure, a stirring unit for stirring the inside of the container and a heating unit such as heater for controlling the temperature of a solution in the container were used assay.

Using the biosensor, the mass of an antibody bound to the surface of the gold electrode of the sensor per unit area is assayed by monitoring the change of the resonance frequency number, using the Sauerbrey equation.

$$\Delta F = - 2F_0{}^2 \Delta m / (A \sqrt{\rho_q \mu_q})$$

$\Delta F$ is the frequency change (Hz) counted; $F_0$ is the fundamental resonance frequency of the quartz oscillator (27 MHz in this Example); $\Delta m$ is the mass change; A is the area of the electrode (0.049 $cm^2$); $\rho_q$ is quartz density (2.65 $gcm^{-3}$); and $\mu_q$ is the shear stress of quartz (2.95 x $10^{11}$ dyn • $cm^{-2}$). According to the equation and the value, the 0.62 ng • $cm^{-2}$ increment of the mass on the sensor surface reduces the frequency by 1 Hz.

In Fig. 1, TNF$\alpha$ 4 was immobilized on the surface of the gold electrode 2 of the sensor; and an assay buffer (PBS and inactivated 15 % fetal calf serum (FCS))

of about 495 $\mu$L was injected into the container 3. The inactivated FCS is a blocking molecule to reduce non-specific binding of the whole blood components on the surface of the electrode. FCS was cultured at about 56°C for about 60 minutes and filtered through a filter of a 0.2-$\mu$m maximum pass-through particle size, for the inactivation.

An infliximab sample 6 at various concentrations was dissolved in a buffer, for example PBST (PBS, 0.1 % Tween 20), or whole blood, to prepare sample solutions 6'.

After waiting for the inactivated FCS bound to the sensor to reach the saturated state, the sample solutions 6' at various concentrations were added at about 5 $\mu$L to the inside of cells 3 filled with an assay buffer 5 of about 495 $\mu$L . In the following Example, the binding rate of infliximab 6 was measured at that time.

[Immobilization of Substance Binding to Sensing Device by Specific Interaction with Object Protein]

Before measurement, the surface of the gold electrode of the sensor was washed with a 1 % SDS solution and the piranha solution ($H_2SO_4$ (30 %) : $H_2O_2$ = 3:1), so as to remove organic contaminants from the surface. After the procedure, the surface of the gold electrode was rinsed several times with distilled water and then left to stand alone in 0.2 M phosphate-buffered physiological saline (PBS) (pH 7.4) in atmosphere at 25°C for 15 minutes.

Using a TNF$\alpha$ solution of about 0.2 $\mu$g/mL, TNF$\alpha$ 4 was immobilized on the gold electrode 2 of the sensor. As a stabilizer, then, bovine serum albumin (BSA) 7 was immobilized together with TNF$\alpha$.

Using then the sensor immobilized with TNF$\alpha$ and BSA and the sensor immobilized only with BSA, frequency change was measured while injecting infliximab at various concentrations.

Specific binding between infliximab and TNF$\alpha$ can be represented by the frequency change depicted by the graph (a) in Fig. 2. The final infliximab concentrations marked with black arrows shown in the figure are 2 ng/mL, 20 ng/mL, 200 ng/mL, 2 $\mu$g/mL, 20 $\mu$g/mL and 40 $\mu$g/mL in the order from the left side.

The graph (a) indicates that the specific binding of infliximab to TNF$\alpha$ was saturated at 20 $\mu$g/mL before reaching 40 $\mu$g/mL, since infliximab of the 40 $\mu$g/mL concentration was at a smaller frequency change than the frequency change of infliximab of the 20 ng/mL concentration.

As shown in the graph (b) in the figure, alternatively, the binding between infliximab and BSA is such that infliximab never specifically binds to BSA. The arrows shown atop in b represent final concentrations of 20 ng/mL, 200 ng/mL, 2 $\mu$g/mL and 20 $\mu$g/mL, in the order from the left side.

[Example 1]

Using cells of a sensor immobilized with TNF$\alpha$

on the surface of the gold electrode, sample solutions (a) to (g) containing infliximab dissolved in PBS and whole blood were injected into the cells to measure frequency change. Individual infliximab concentrations of the sample solutions were (a) 0 μg/mL, (b) 5 μg/mL, (c) 10 μg/mL, (d) 30 μg/mL, (e) 50 μg/mL and (g) 100 μg/mL. About 5 μL of each of the sample solutions was injected into an assay buffer of about 495 μL.

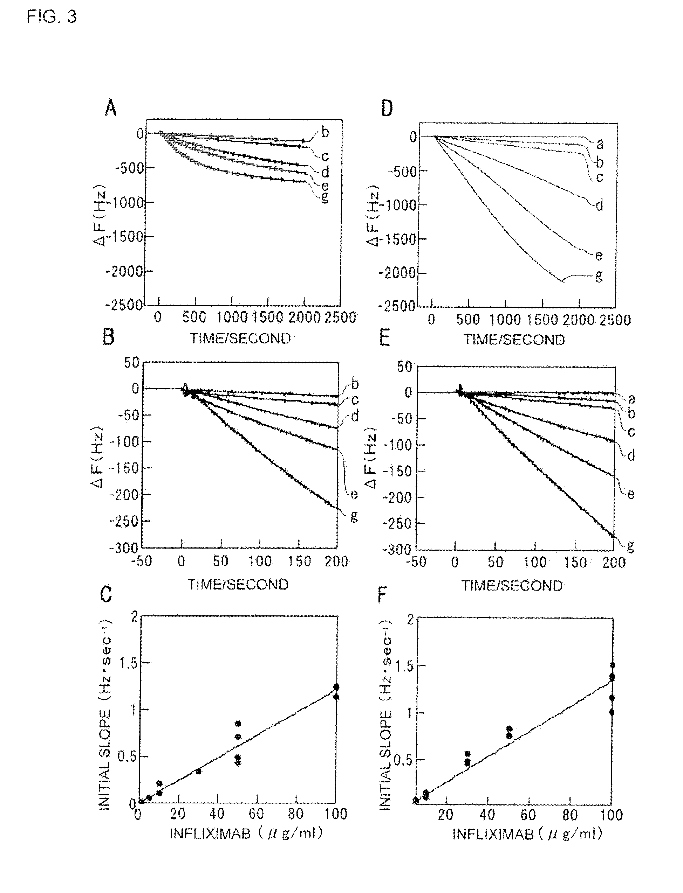

Figs. 3A to 3C show the results of the measurement of the specific binding of infliximab to TNFα which are obtained by injecting the sample solutions (a) to (g) in PBS.

The frequency change at the concentrations of the individual sample solutions is shown in Fig. 3A, while an enlarged view of Fig. 3A from 0 to 200 seconds is shown in Fig. 3B.

Fig. 3B indicates that a binding reaction represented by a linear frequency change occurred within 100 seconds of the start of injecting the sample solutions containing infliximab. At the concentrations of the sample solutions (b) to (g), the initial binding rate is represented by the graph slope.

Fig. 3C shows plots of initial binding rates at 1 to 100 μg/mL infliximab concentrations. It is herein shown that the initial binding rate and the infliximab concentration are in a linear relation.

The dissociation constant of infliximab bound to TNFα and the rate parameter can be measured by curve regression analysis of the individual binding curves by an expression represented by 1:1 binding model.

$K_d$ = 0.48 nM was obtained, which was very close to the dissociation constant (0.45 nM) of infliximab from the transmembrane TNFα, as reported previously.

Figs. 3D to 3F show the results of the measurement of the specific binding of infliximab to TNFα by injecting sample solutions (a) to (g) dissolved in whole blood.

The frequency change of the individual sample solutions is shown in Fig. 3D, while an enlarged view of Fig. 3D from 0 to 200 seconds is shown in Fig. 3E.

Unlike Figs. 3A to 3C, the binding reaction of infliximab to the sample solutions dissolved in whole blood was at the final frequency change of 3 fold, while the curves of the binding reaction could not be analyzed by using a theoretical curve in the 1:1 binding model. This indicates that whole blood components and the infliximab-TNFα complex bind together multiply on the surface of the sensor.

Fig. 3E shows the state of initial binding. As shown in Fig. 3F, a linear binding reaction was obtained even in samples of infliximab dissolved in whole blood on plots of initial binding rates vs. infliximab concentrations within 1 to 100 μg/mL.

[Example 2]

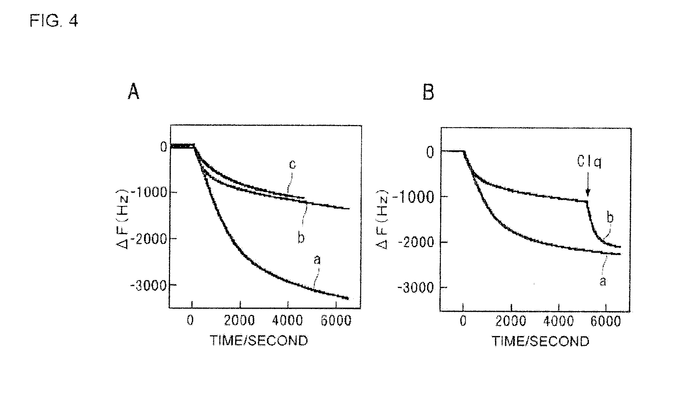

The method for assaying the binding of a complement-based protein to the infliximab-TNFα complex is described with reference to Fig. 4.

TNFα is immobilized on the surface of the gold electrode of the sensor. 100 μg/mL infliximab was dissolved individually in whole blood and a plasma solution with a thermally inactivated complement system, to prepare sample solutions of infliximab dissolved in whole blood and sample solutions of infliximab dissolved in inactivated plasma.

5 μL of the individual sample solutions was injected in an assay buffer of 495 μL for measurement, and the results are shown in Fig. 4A. Herein, graph a shows frequency change in whole blood while graph b shows frequency change in inactivated plasma. Fig. 4A indicates that graph b is at a smaller frequency change than the frequency change of graph a.

5 μL of sample solutions of infliximab dissolved in whole blood was injected in a PBS (15 % FCS) assay buffer of 495 μL containing 5 mM EDTA for measurement. Because EDTA works as a substance suppressing the formation reaction of the primary C1 complex, graph c representing infliximab dissolved in whole blood in the PBS (15 % FCS) assay buffer containing 5 mM EDTA is at a smaller frequency change, compared with graph a representing infliximab dissolved in whole blood in the assay buffer without EDTA.

These results indicate that the multiple binding of infliximab dissolved in whole blood occurs from the multiple binding of the protein of the complement system in whole blood.

[Example 3]

So as to examine that the complement protein can bind to the infliximab-TNFα complex on the sensor surface in a secure manner, an example of the measurement of C1q binding to the infliximab-TNFα complex is described with reference to Fig. 4B.

5 μL of sample solutions of 100 μg/mL C1q and 100 μg/mL infliximab dissolved in PBS was injected in an assay buffer of 495 μL for measurement in cells, and the results are shown on graph a. 5 μL of sample solutions of 100 μg/mL infliximab dissolved in PBS was injected in an assay buffer of 495 μL for measurement in cells; after the binding of infliximab to TNFα on the sensor surface reached the saturation state, 5 μL of 100 μg/mL C1q was added for measurement, and the results are shown in graph b. Graphs a and b indicate that the binding ratios within 200 seconds after the start of the measurement were almost identical.

As the subsequent binding reaction, the frequency change representing the binding of the sample solutions of infliximab and C1q dissolved in PBS was about 2,000 Hz. This was higher by about 1,000 Hz than the frequency change of the sample solution of infliximab alone dissolved in PBS.

The results show that C1q bound to TNFα on the sensor surface after the binding of infliximab to TNFα on the sensor surface reached the saturation state (about 5,000 seconds).

[Evaluation of Protein-based Drug]

The complex formation rate of infliximab and TNF$\alpha$ is represented by $k_{on}$ [infliximab] [TNF$\alpha$] - $k_{off}$ [infliximab/TNF$\alpha$], using binding rate ($k_{on}$), dissociation rate ($k_{off}$) and individual molecule concentrations (concentration of each molecule is represented above by [molecule name]). By deleting the second term, the rate can be simplified as $k_{on}$ [infliximab] [TNF$\alpha$] , at the initial binding period. The reason is that the value of [infliximab/TNF$\alpha$] is small during the initial binding. Therefore, the change of the initial frequency within 100 seconds from the start of the measurement can be represented by a linear expression representing infliximab concentration. The slope defining the initial binding rate can be obtained. The initial binding rate is in proportion to the infliximab concentration.

The initial binding rate of infliximab at concentrations of 1 to 100 $\mu$g/mL in sample solutions of infliximab dissolved in PBS and of infliximab dissolved in whole blood is in linear relation. The pharmacokinetics research works of infliximab show that the median of the peak infliximab concentration is about 90 to 110 $\mu$g/mL after 5 mg/kg dosing three times daily for 8 weeks. The serum trough concentration of infliximab is about 1 $\mu$g/mL. Therefore, the dynamic range of the method includes the appropriate concentration range for the measurement of the blood concentration of infliximab during therapeutic treatment.

Compared with other methods for assaying antibodies like ELISA which require a far longer time and complicated experimental steps, protein-based drugs can be rapidly assayed with whole blood within 100 seconds, in accordance with the invention, with no need of protein modification, enzyme amplification or dilution or centrifugation. The invention is comparatively simple as a technique and has an ability to evaluate drug concentrations within several minutes. Compared with current methods for assaying protein-based drugs, that point is a significant advantage.

The method is applicable to an assay of almost all of protein-based drugs such as human antibodies, murine antibodies and fusion proteins, in addition to chimera antibodies. For example, etanercept is a fusion protein of the Fc component of human immunoglobulin G1 and the human soluble TNF$\alpha$ receptor and has been approved by FDA as a therapeutic drug of rheumatoid arthritis. Compared with the affinity of infliximab, the affinity of etanercept bound to TNF$\alpha$ is slightly poor, but the conditions of the buffer solution and the volume of the sample injected were optimized for analyzing etanercept. The measurement of etanercept within 1 to 100 $\mu$g/mL in whole blood is achieved. Similarly, adalimumab is a complete human antibody. It is considered that adalimumab is at a lower immunogenicity level than the immunogenicity levels of chimera antibodies, but recent survey reports tell that the occurrence of anti-adalimumab antibodies reduce not only the clinical reaction of the therapeutic drug but also the drug concentration. The method can be optimized for the assay of the blood concentration of adalimumab, like other therapeutic drugs of human antibodies.

The inventive method can be used for the evaluation of therapeutic drugs such as ibritumomab tiuxetan and gemutuzumab ozogamicin. Such therapeutic drugs involve the use of antibodies fused with other biologically active substances such as chemotherapeutic drugs or isotopes. By preparing a sensor surface carrying a ligand complementing the antibody element of a drug, the method can be used so as to assay an antibody with some relation by using a similar method.

[Evaluation of Complement-dependent cytotoxicity Activity]

The inventive method may be applicable to the measurement of the severity of diseases and therapeutic reactions, because the method is capable of sensing and measurement of multiple binding reactions including TNF$\alpha$ and complements. Abnormal CDC is a significant element for the onset of many autoimmune diseases; and the measurement of abnormal CDC enables the measurement of individual difference in the severity of diseases. Furthermore, transmembrane TNF$\alpha$ is an important target for complement-dependent cytotoxicity (CDC) which involve the binding of complement protein complexes to cells with transmembrane TNF$\alpha$ for the action, using the mechanism to be used in removing cells generally undesirable for human bodies. Monoclonal antibody-based drugs of a certain specific type, such as rituximab, modulate the CDC activity in particular. It can be expected that other therapeutic drugs to reduce TNF$\alpha$, such as infliximab, may have the same effect. To assay the level of the CDC activity is an objective scale for assaying the progress of a disease or a therapeutic reaction.

The method herein described can assay rapidly antibody-based drugs through the analysis of the initial binding profile, while the same method can assay the CDC activity. The level of the CDC activity can be determined by subtracting the results of a control experiment with inactivated complement activity from experimental results and comparing the resulting value with the known standard value. To doctors consulted by patients, the rapid method for evaluating the CDC activity can provide advantageous data about clinical effects of such therapeutic drugs and indicators for thereby determining a therapeutic method.

Additionally, the sensor as a sensing device used in the measurement can measure a great number of samples simultaneously, when a plurality of the sensor is used. By immobilizing another protein to another sensor, the concentration of the protein can be measured.

The method for calculating the measured results includes for example but is not limited to a method for calculating the concentration of a protein to be measured in blood on the basis of the mass of an antibody existing on the sensor surface per unit area.

Industrial Applicability

As described above, in accordance with the invention, the amount of a protein-based drug circulating in a patient body can be measured, which enables wide industrial applications such as the determination of an appropriate dose of the drug, the evaluation of the severity of a disease and autoimmunity to the disease, or the evaluation of a patient reaction to the therapeutic treatment using the protein-based drug.

Description of Symbols

1     Quartz plate
2     Electrode
3     Container
4     TNF$\alpha$
5     Assay buffer
6     Infliximab
6'     Sample solution
7     BSA

**Claims**

1. A method for quantification of the body internal concentration of a protein-based drug, comprising the following steps:

   step A comprising using a sensing device mounted with a substance binding to a protein contained in the protein-based drug through a specific interaction to determine the binding mass per unit area of a solution containing the protein-based drug to the substance mounted on the sensing device as a standard value; and step B comprising assaying a collected biological sample by the sensing device and comparing the resulting value with the standard value to determine the concentration of the protein-based drug contained in the biological sample.

2. A method for quantification of the body internal concentration of a protein-based drug according to claim 1, wherein the biological sample is whole blood containing clot and serum.

3. A method for quantification of the body internal concentration of a protein-based drug according to claim 1, wherein the biological sample is plasma or serum.

4. A method for quantification of the body internal concentration of a protein-based drug according to claim 1, wherein the standard value at the step A is used as a fixed standard value preliminarily assayed at the step A, using a sample solution containing the protein-based drug or wherein the step A is simultaneously carried out with the step B or the step A is carried out within 24 hours before the step B.

5. A method for quantification of the body internal concentration of a protein-based drug according to claim 1, wherein the sensing device is any of a quartz oscillator, a surface plasmon resonance device and an interferometer.

6. A method for quantification of the body internal concentration of a protein-based drug according to claim 1, wherein the protein is any of a monoclonal antibody, a chimera monoclonal antibody, a humanized monoclonal antibody, a human monoclonal antibody and a murine monoclonal antibody and a fusion protein containing an antigen-binding site of an antibody and a fusion protein containing an antigen-binding site of a receptor.

7. A method for quantification of the body internal concentration of a protein-based drug according to claim 1, wherein a ligand binding to the protein via a specific interaction is immobilized on the sensing device.

8. A method for quantification of the body internal concentration of a protein-based drug according to claim 1, wherein the specific interaction is antibody-antigen receptor binding or ligand-receptor binding.

9. A method for quantification of the body internal concentration of a protein-based drug according to claim 1, wherein the body internal concentration of the protein-based drug is used for determining an appropriate dose of the drug.

10. A method for quantification of the body internal concentration of a protein-based drug according to claim 1, comprising using a monoclonal antibody-based drug as the protein-based drug; and assaying a complement-based protein interaction occurring via the binding of the monoclonal antibody-based drug to the sensing part of a sensing apparatus at an active state of a complement in the biological sample and assaying an interaction of the monoclonal antibody-based drug at an inactive state of the complement in the biological sample and determining on the basis of the difference between the two assay values that the complement level is high in a manner corresponding to the cytotoxicity.

FIG. 1

FIG. 2

FIG. 3

FIG. 4

## INTERNATIONAL SEARCH REPORT

| | International application No. |
|---|---|
| | PCT/JP2010/000198 |

### A. CLASSIFICATION OF SUBJECT MATTER
*G01N33/543*(2006.01)i

According to International Patent Classification (IPC) or to both national classification and IPC

### B. FIELDS SEARCHED

Minimum documentation searched (classification system followed by classification symbols)
G01N33/53-579

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII), CAplus(STN)

### C. DOCUMENTS CONSIDERED TO BE RELEVANT

| Category* | Citation of document, with indication, where appropriate, of the relevant passages | Relevant to claim No. |
|---|---|---|
| X/A | JP 2009-500623 A (WYETH),<br>08 January 2009 (08.01.2009),<br>claims; paragraphs [0004], [0009] to [0016],<br>[0042], [0027] to [0030], [0037], [0038],<br>[0056]<br>& US 2007/0003559 A1     & EP 1899731 A<br>& WO 2007/005690 A1     & CA 2613880 A<br>& NO 20080216 A          & KR 10-2008-0026206 A<br>& CN 101253408 A | 1-9/10 |
| A | WO 2008/041594 A1 (Olympus Corp.),<br>10 April 2008 (10.04.2008),<br>(Family: none) | 1-10 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 April, 2010 (02.04.10) | 13 April, 2010 (13.04.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61144910 A
- JP 2003535594 T